# EUROPEAN PATENT APPLICATION

(11) **EP 3 730 116 A1**
(43) Date of publication of application: **28.10.2020**
(21) Application number: 18891908.8
(22) Date of filing: 24.10.2018
(51) Int. Cl.: A61H 1/00, A61H 1/02, A61B 5/103, A61B 5/11, A61F 5/01

(54) **SCOLIOSIS CORRECTION APPARATUS**

(30) Priority: 18.12.2017 KR 20170173743
(71) Applicant: Moon, Yang Gyu, Ulsan 44923 (KR); Lee, Jung Woo, Ulsan 44923 (KR)
(72) Inventor: Moon, Yang Gyu, Ulsan 44923 (KR); Lee, Jung Woo, Ulsan 44923 (KR)
(74) Representative: Zacco Sweden AB
(86) International application number: PCT/KR2018/012644
(87) International publication number: WO 2019/124714

(57) **Abstract**

The present invention relates to an apparatus that adjusts equilibrium of a body via a footboard that may be adjusted vertically and left-to-right, and corrects scoliosis via same. The inclination angle of the footboard is adjusted fully automatically, and thus may be adjusted to a fine angle, thereby providing a customized angle suited to characteristics of a user. Consequently scoliosis may be corrected by a proper standing posture and standing motion, and because the footboard inclines left-to-right, imbalances of the legs such as bowlegs may be corrected in addition to scoliosis.

## Description

### Field

The present disclosure relates to a scoliosis correction apparatus, and relates to an apparatus for correcting scoliosis by adjusting a balance of a body using a foot support that may tilt in up and down and left and right directions.

### Description of Related Art

A normal spine of a human body is straight when viewed from a front. When viewed from a side, a cervical vertebrae and a lumbar vertebrae of the normal spine are curved forwards, and a thoracic vertebrae and a sacral vertebrae thereof are curved backwards.

Since modern people spend a lot of time in a sit state, there is a greater possibility of having a bent spine due to a wrong posture or habit. In particular, adolescents in a growing stage are more affected by a posture because bones thereof are more flexible than bones of adults who have stopped growing. When the spine is bent into a C-shape or an S-shape and thus a body is tilted or turned to a left or right side. This is called scoliosis.

Such scoliosis may not be detected in an early stage because subjective symptom thereof does not appear in the early stage. As the scoliosis progresses, muscles, ligaments, and nerves around the spine are compressed such that symptoms such as low back pain, stiff shoulder, and headache occur. As the progression further progresses, deformation of the spine into ribs and pelvis may occur, thereby compressing a heart and a lung, such that abnormal cardiopulmonary function may occur. Further, some patients have severe pain during sleep. At a middle age at which a gap between discs is reduced, fatigue may easily occur due to skeletal and bone imbalances and she or he may not maintain a constant posture for a long time, and may have headache and shoulder pain. The scoliosis may lead to imbalance of hormones and digestive functions. The scoliosis may lead to allergies, concentration disorders, and growth disorders in adolescent patients.

Thus, the scoliosis must be corrected. In order to correct the scoliosis, Korean Registered Utility Model No. 0,482,054 discloses a scoliosis correction device to allow a user to adjust an inclination angle so that the user performs exercises including stretching, or waist exercise to straighten the spine to correct the posture.

However, in the above scoliosis correction device, the user may adjust the inclination angle step by step by himself or herself, and therefore, may not adjust the inclination angle suitable for the user in a fine manner, and the device may not realize customized correction suitable for a body shape of the user.

### DISCLOSURE

### TECHNICAL PURPOSES

A purpose of the present disclosure is to provide a scoliosis correction apparatus to automatically adjust an inclination angle of a foot support to allow a user to straighten a spine to correct a posture thereof.

Further, another object of the present disclosure is to provide a scoliosis correction apparatus to allow the foot support to tilt in a left or right direction to correct imbalance of a lower body such as bowleg.

### TECHNICAL SOLUTIONS

One aspect of the present disclosure provides a scoliosis correction apparatus comprising: a pair of foot supports to measure a body weight, a distribution of the body weight and a foot pressure of a user; a support adjuster hinge-coupled to a front end of each of the foot supports and having a ball groove defined in a lower portion thereof; an angle adjuster including a main actuator cylinder having one end received in the ball groove to push or pull the support adjuster to adjust a vertical level of the front end of each foot support to adjusts an front-rear direction inclination angle of each foot support; and a controller configured to determine scoliosis and posture imbalance of the user based on the body weight, the distribution of the body weight, and the foot pressure measured by the foot supports and to control the angle adjuster based on the determination result.

In one embodiment, the angle adjuster further includes an auxiliary actuator cylinder coupled to a side face of each foot support and disposed outside the main actuator cylinder.

In one embodiment, the auxiliary actuator cylinder extends in a vertical direction while the main actuator cylinder extends in a horizontal direction.

In one embodiment, the main actuator cylinder has a pushing head inserted into the ball groove of the support adjuster, wherein a first male thread is formed on an outer face of a front end of the pushing head, and a second male thread is formed on an outer face of a middle portion of the pushing head.

In one embodiment, a first female thread corresponding to the first male thread of the pushing head is formed on an inlet of the ball groove, wherein a second female thread corresponding to the second male thread is formed on an innermost portion of the ball groove.

In one embodiment, at least one ceramic ball is formed on a top face of the foot support.

In one embodiment, the scoliosis correction apparatus further comprises a support base for supporting the foot supports, the support adjuster, the angle adjuster, and the controller thereon, wherein the support base has a load cell configured to measure a weight of the user excluding weights of the foot support, the support adjuster, the angle adjuster, and the controller.

In one embodiment, the scoliosis correction apparatus further comprises: a stereoscopic camera extending from the support base in a vertical direction to scan a body of the user and measure a center point of each of skeletons of the user; and a laser irradiator for irradiating a first laser beam to an actual center point of each skeleton of the user and a second laser beam to a central point of each skeleton as a reference point for correction, thereby to induce a target posture of the user.

In one embodiment, the scoliosis correction apparatus further comprises: a handle disposed at an upper portion of the post and moving in up and down and left and right directions and rotating around the post; and a display connected to the handle, and moving up and down according to the up and down movement of the handle, wherein the display visually present measurements, determinations, and instructions from the controller to the user.

In one embodiment, each foot support has a plurality of acupressure protrusions projecting from a top face of the foot support and is capable of vertical movement.

### TECHNICAL EFFECTS

According to the present disclosure, the inclination angle of the foot support is automatically adjusted in a fine manner, thereby providing a customized angle suitable for characteristics of the user to allow a correct standing posture and a correct standing movement to correct scoliosis.

Further, according to the present disclosure, the foot support tilts in a left or right direction to correct leg imbalance such as bowleg in addition to correction of the scoliosis.

### BRIEF DESCRIPTION OF THE DRAWINGS

FIG. 1 is a perspective view of a scoliosis correction apparatus according to the present disclosure.
FIG. 2 is a side view of a scoliosis correction apparatus according to the present disclosure.
FIG. 3 is a cross-sectional view of a foot support according to another embodiment of the present disclosure.
FIG. 4 is a cross-sectional view of a foot support according to another embodiment of the present disclosure.
FIG. 5 is an example of a combination of a pushing head and a ball groove according to the present disclosure.
FIG. 6 is a side view of a scoliosis correction apparatus according to another embodiment of the present disclosure.
FIG. 7 is a perspective view of a scoliosis correction apparatus according to another embodiment of the present disclosure.
FIG. 8 is a perspective view of a scoliosis correction apparatus according to another embodiment of the present disclosure.
FIG. 9 is a perspective view of a scoliosis correction apparatus according to another embodiment of the present disclosure.

### DETAILED DESCRIPTIONS

Hereinafter, an embodiment of the present disclosure will be described in detail with reference to the drawings.

However, the present disclosure is not limited to the embodiments disclosed below, but will be implemented in various different forms. These embodiments are provided only to allow the present disclosure to be complete, and fully inform the skilled person to the art of a scope of the disclosure. The same reference numerals in the drawings refer to the same elements.

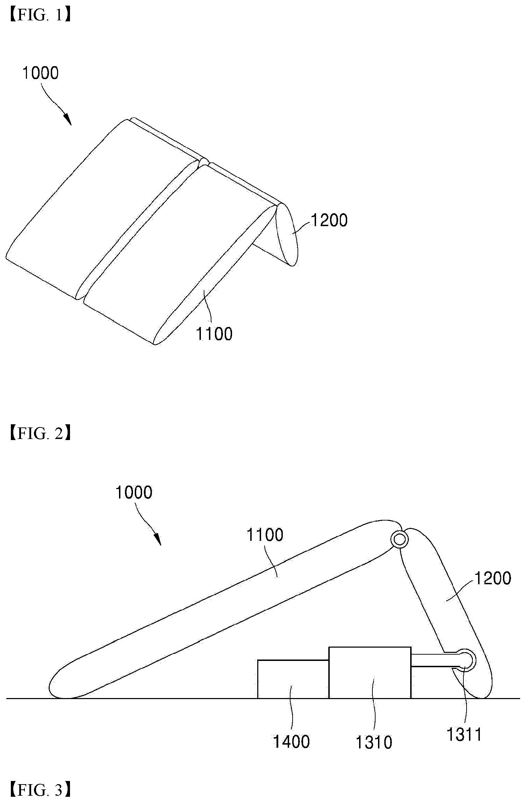

FIG. 1 is a perspective view of a scoliosis correction apparatus according to the present disclosure, and FIG. 2 is a side view of a scoliosis correction apparatus according to the present disclosure.

Referring to FIG. 1 and FIG. 2, a scoliosis correction apparatus according to the present disclosure includes a pair of foot supports 1100 to measure a user's body weight, a distribution of the body weight and a foot pressure, a support adjuster 1200 having a rear end hinge-coupled to a front end of each of the foot supports 1100, and having a ball groove 1210 defined in a lower portion thereof, and an angle adjuster 1300 including a main actuator cylinder 1310 having one end inserted into the ball groove of the support adjuster 1200 to push the support adjuster 1100 such that the front end of each of the foot supports 1100 moves up and down to adjust an inclination angle of the foot supports 1100, and a controller 1400 that determines scoliosis and posture imbalance of a user, based on the body weight, the body weight distribution, and the foot pressure measured at the foot supports 1100, and to control the angle adjuster 1300 to adjust the inclination angle of the foot supports 1100.

Each foot support 1100 is a plate-shaped member that the user's foot contacts and measures the body weight as a load exerted by the user when the user's foot touches the support, and a load and a foot pressure exerted by each foot (each of left foot and right foot). In this connection, the foot pressure refers to a contact surface and a pressure applied to a sole portion of the user.

To this end, the foot support 1100 is preferably an arched member. When the support 1100 is a flat member, an area in which the support does not come into contact with the user's foot is large, whereas when the support is an arched member, the support has a maximum contact with the user's foot arch to make a determination on a larger area. Further, as the contact area increases, friction also increases, so that sliding of the sole of the foot due to the angular deformation of the foot support may be prevented.

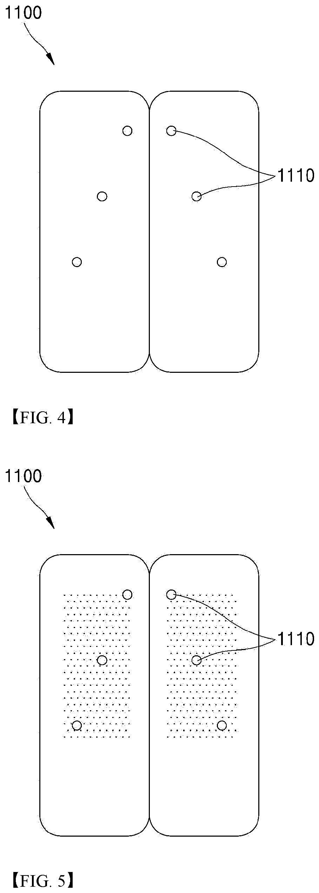

FIG. 3 and FIG. 4 are cross-sectional views of the foot supports 1100 according to another embodiment of the present disclosure.

Referring to FIG. 3 and FIG. 4, each foot support 1100 may include at least one ceramic balls 1110 has a convex hemisphere shape on a top face thereof contacting the sole of the user. The ceramic ball 1110 radiates far infrared ray to bring about a warming effect due to resonance of the body. In other words, when the far-infrared ray contacts water, vibration occurs. More than 70% of the human body is composed of water, and thus blood circulation is accelerated due to the vibration resulting from the far infrared rays, resulting in a warm-blood effect.

In this connection, the ceramic ball 1110 is fixed to the top face of the foot support 1100, but may rotate. Emissivity of the far-infrared radiation as emitted from the ceramic ball 1110 is improved due to frictional heat generated when the ceramic ball 1110 rotates while being in contact with the sole of the foot, thereby having a greater warm-blood effect.

Further, the ceramic ball 1110 may be used as a reference point based on which the user correctly positions the user's foot on the top face of the foot support 1100. For example, when one ceramic ball 1110 is formed, the ball may be used as a guideline to indicate a location of a specific toe and a heel of the user. When a plurality of the ceramic ball 1110 are arranged, the foot must be positioned within the arrangement region of the ceramic balls 1110. Accordingly, resonance may be caused in various parts of the sole.

In this connection, a plurality of fine protrusions may be formed on the top face of the foot support 1100. The fine protrusions may ascend or descend. The controller may determine a location of each reflector according to a foot size based on the user's foot measured and scanned at foot support 1100, and the protrusion ascends to acupressure the sole of the foot based on each reflector. In this connection, the protrusions may not ascend at the same time but may ascend by a certain range in a state where the user is standing on the foot support 1100 within a range where the posture is not deformed so that the purpose of the present disclosure is not lost. The protrusion as close as possible to one reflector ascends. This may use a lightly friction message scheme.

Further, the rear end of the support adjuster 1200 is hinged-coupled to the front end of the foot support 1100. The ball groove 1210 is formed in a lower portion of the support adjuster.

The ball groove 1210 may receive a distal end of the main actuator cylinder 1310 of the angle adjuster 1300 to be described later. The ball groove 1210 includes, at an inlet thereof, a second female thread 1211a, and, at the innermost portion thereof, a first female thread 1211b.

One end of the angle adjuster 1300 is inserted into the ball groove of the support adjuster 1200, thereby pushing the support adjuster 1200. Since the front end of the foot support 1100 moves up and down when the support adjuster 1200 is pushed, the inclination angle of the foot support 1100 may change according to a distance by which the angle adjuster 1300 is pushed.

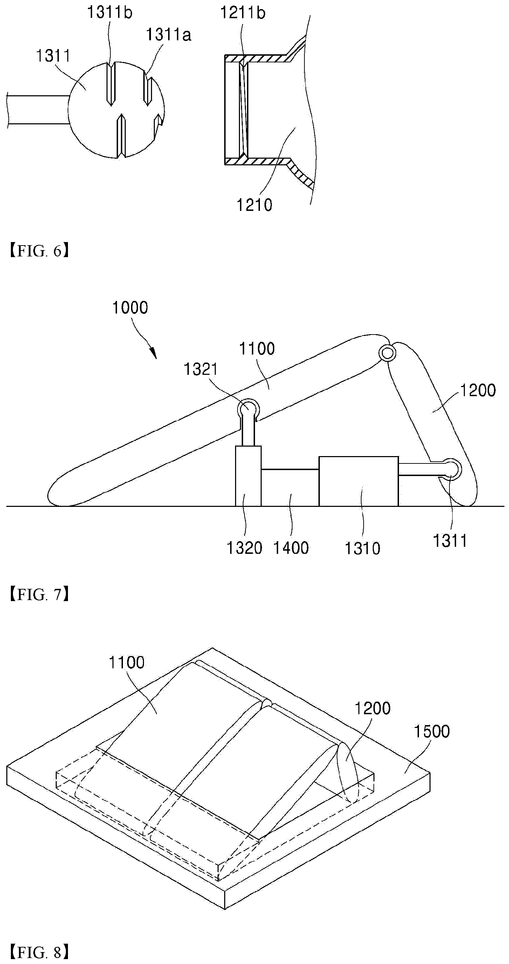

FIG. 5 is an exemplary diagram showing a combination of a pushing head 1311 and the ball groove 1210 according to the present disclosure. Referring to FIG. 5, one end of the angle adjuster 1300 is inserted into the ball groove 1210, and the angle adjuster 1300 includes the pushing head 1311 which has the same shape as the ball groove 1210. The head is inserted into the groove. The pushing head 1311 has a shape corresponding to the ball groove 1210, but a maximum outer diameter of the pushing head 1311 is the same as an inner diameter of the inlet of the ball groove 1210.

To this end, a first male thread 1311a corresponding to the first female thread 1211b of the ball groove 1210 is formed on a front end of the pushing head 1311. A second male thread 1311b corresponding to the second female thread 1211b of the ball groove 1210 is formed in a middle portion of the pushing head 1311.

In this connection, the pushing head 1311 of the main actuator cylinder 1310 is not pushed toward the inside of the ball groove 1210 in a mere linear manner, but is inserted into the inside of the ball groove 1210 while rotating at the inlet of the ball groove 1210. That is, the head may rotate while the second male thread 1311b located at the middle portion of the pushing head 1311 is engaged with the second female thread 1211b of the ball groove 1210. When the second male thread 1311b and the second female thread 1211b are engaged with each other, the support adjuster 1200 and the angle adjuster 1300 according to the present disclosure are in a fixed state. However, when the pushing head 1311 continuously rotates, the second male thread 1311b of the pushing head may be disengaged with the female thread 1211b and thus may be inserted into the ball groove 1210.

FIG. 6 is a side view of a scoliosis correction apparatus according to another embodiment of the present disclosure.

Referring to FIG. 6, the angle adjuster 1300 according to the present disclosure may further include an auxiliary actuator cylinder 1320 located outside the main actuator cylinder 1310 and coupled to a side face of the foot support 1100.

The auxiliary actuator cylinder 1320 extends in a direction perpendicular to an extension direction of the main actuator cylinder 1310, and is connected to the foot support 1100. The auxiliary actuator cylinder 1320 distributes a load applied to the foot support 1100, thereby preventing damage thereto.

To this end, the foot support 1100 further includes a ball groove defined in a side face thereof. The ball groove of the foot support 1100 receives one end of the auxiliary actuator cylinder 1320 and has threads at an inlet and the innermost portion of the ball groove. The threads correspond to threads formed on a front end and a middle portion of the auxiliary actuator cylinder 1320. A method of engaging the auxiliary actuator cylinder 1320 and the foot support 1100 with each other is the same as the method of engaging the support adjuster 1200 and the angle adjuster 1300 with each other as described above, and thus detailed descriptions thereof are omitted.

However, the auxiliary actuator cylinder 1320 is fixed to the side face of the foot support 1100. When the auxiliary actuator cylinder 1320 descends, the side face of the foot support 1100 is inclined toward a ground. Further, when the auxiliary actuator cylinder 1320 ascends, the side face of the foot support 1100 ascends and inclines upwards. Thus, the vertical movement of the auxiliary actuator cylinder 1320 may tilt the foot support 1100 in a left or right direction to correct the imbalance of the leg such as the bowleg.

The controller 1400 determines the scoliosis and the posture of the user, based on the body weight, the body weight distribution, and the foot pressure measured by the foot support 1100 and controls the angle adjuster 1300 to adjust the inclination angle of the foot support 1100 so that the user's posture may be corrected.

In one example, the controller 1400 stores a distribution of left and right body weights and the foot pressure measured by foot support 1100 as first data, and controls the angle adjuster 1300 based on the first data. Thus, the inclination angle of the foot support 1100 may be adjusted. In this connection, the controller 1400 continuously stores the left and right body weight distribution and the foot pressure of the user as second data and third data. The stored data are compared with each other upon the adjustment of the inclination angle by the angle adjuster 1300 to detect a moment when the user's left and right body weight distribution and foot pressure suddenly change. The detected moment may be a critical point when the user's posture may be maintained. Thus, the controller 1400 may stop the operation of the foot support 1100 at the inclination angle corresponding to the critical point and maintain the stop state and then may repeat increasing and decreeing operations of the inclination angle. In this way, the user's posture is adjusted and corrected via the repetition.

FIG. 7 is a perspective view of a scoliosis correction apparatus according to another embodiment of the present disclosure.

Referring to FIG. 7, a scoliosis correction apparatus according to another embodiment of the present disclosure further includes a support base 1500 for support the foot support 1100, the support adjuster 1200, the angle adjuster 1300 and the controller 1400 thereon. In this connection, the foot support 1100 does not measure the weight of the user. The user's weight may be measured via a load cell included in the support base 1500. When the foot support 1100 performs various functions on the same area, a volume may be increased. Thus, the user's body weight may be measured by the support base 1500, in consideration of the volume increase. When the user's body weight may be measured by the support base 1500, the support base may exclude weights of the foot support 1100, the support adjuster 1200, the angle adjuster 1300 and the controller 1400 located on the top of the support base 1500. Thus, only a weight resulting from the user standing up on the foot support 1100 may be measured.

Further, the support base 1500 according to the present disclosure further includes a gyro sensor therein to prevent an erroneous result due to the tilt of the scoliosis correction apparatus when the ground is not flat. Therefore, the slope of the ground may be measured by the gyro sensor, thereby to produce a more accurate result via the adjustment of the tilt.

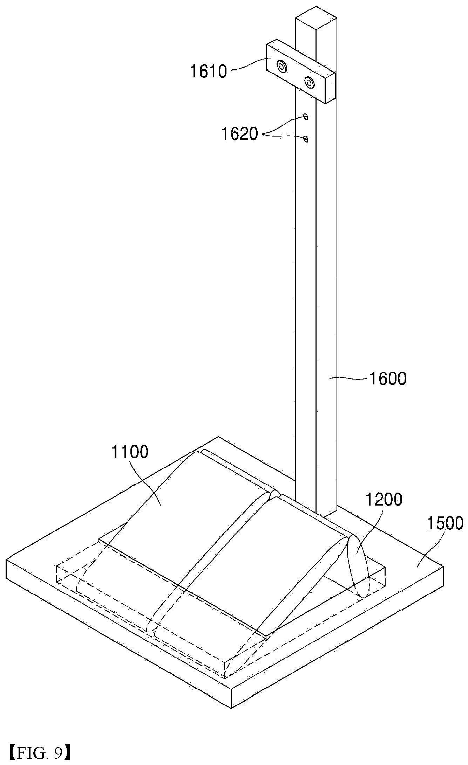

FIG. 8 is a perspective view of a scoliosis correction apparatus according to another embodiment of the present disclosure.

Referring to FIG. 8, a scoliosis correction apparatus according to another embodiment of the present disclosure further includes a post 1600 extending in a vertical direction from the support base 1500. The post 1600 includes a stereoscopic camera 1610 that scans the user's body, and a laser irradiator 1620 that irradiates a reference point for a reference posture with laser to allow the user to maintain a reference posture of the user's body.

The stereoscopic camera 1610 may have two lenses to capture the body of the user, and obtain two images at the same time. The stereoscopic camera 1610 may calculate the reference point for the reference posture based on a skeleton of the body via the stereoscopic vision of the acquired images. This reference point is not acquired one-time, but continuous tracking thereof is performed so that a center point of the skeleton is consistent with the user's body.

The laser irradiator 1620 includes a first laser to irradiate first laser in a connecting manner between the user's skeletal center points measured by the camera, and a second laser to irradiate second laser, based on a target posture determined based on the user's body weight, the body weight distribution and the foot pressure determined by the controller and the user's body image from the stereoscopic camera 1610. The first and second laser beams are irradiated onto the body of the user at the same time. Therefore, the user visually recognizes her/his body imbalance based on the two laser beams irradiated thereto and corrects the posture so that the two laser beams coincide with each other.

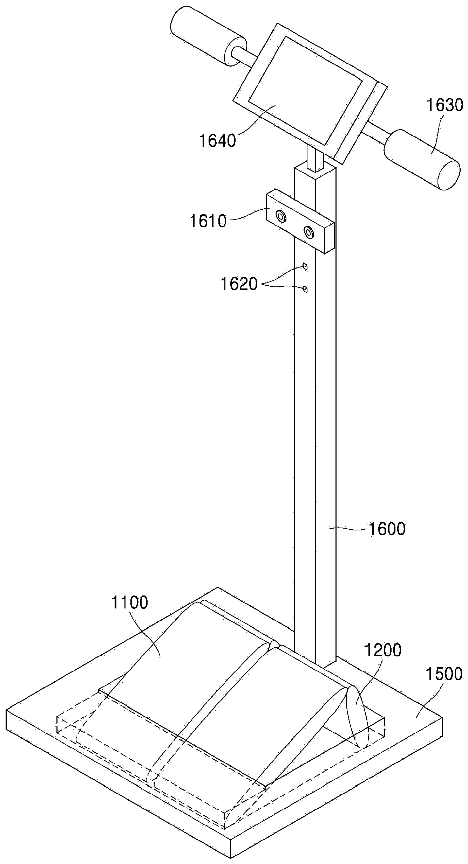

FIG. 9 is a perspective view of a scoliosis correction apparatus according to another embodiment of the present disclosure.

Referring to FIG. 9, a scoliosis correction apparatus according to another embodiment of the present disclosure includes a handle 1630 which moves vertically and horizontally and is disposed at an upper portion of the post 1600, and rotates around the post 1600, and a display 1640 that is located above the handle 1630, and moves up and down according to the vertical movement of the handle 1630 and visually presents the measurement, the determination, and instructions from the controller 1400 and the body skeleton measured by the stereoscopic camera 1610 to the user.

The handle 1630 is used as an aid when it is difficult for the user to balance the posture due to a steep slope of the foot support 1100, or when the user's posture is to be rapidly corrected using a steep slope. Therefore, the handle 1630 may move up and down to be adapted to a height of the user. The handle may rotate to the left and right in order to solve the body imbalance and for stretching. In a preferred embodiment, it is preferable that the handle rotates around the post 1600 in a parallel manner to the ground. Depending on the characteristics of the user, the handle may rotate around the post 1600 toward the body of the user, and thus may be positioned on the same line as an arm of the user holding the handle 1600.

The display 1640 may be connected to the top of the handle 1630 and may move up and down according to the vertical movement of the handle 1630. However, the display does not respond to the rotation of the handle. Further, the measured and recorded body weight of the user, the body weight distribution and the foot pressure are visually displayed on the display 1640. The user's skeleton as measured via the stereoscopic camera1610 of the post 1630 is visually displayed thereon.

Although the present disclosure is described above with reference to the drawings and embodiments, those skilled in the art will understand that the present disclosure may be variously modified and changed within the scope described in the following claims.

## Claims

1. A scoliosis correction apparatus comprising:
a pair of foot supports to measure a body weight, a distribution of the body weight and a foot pressure of a user;
a support adjuster hinge-coupled to a front end of each of the foot supports and having a ball groove defined in a lower portion thereof;
an angle adjuster including a main actuator cylinder having one end received in the ball groove to push or pull the support adjuster to adjust a vertical level of the front end of each foot support to adjusts an front-rear direction inclination angle of each foot support; and
a controller configured to determine scoliosis and posture imbalance of the user based on the body weight, the distribution of the body weight, and the foot pressure measured by the foot supports and to control the angle adjuster based on the determination result.

2. The scoliosis correction apparatus of claim 1, wherein the angle adjuster further includes an auxiliary actuator cylinder coupled to a side face of each foot support and disposed outside the main actuator cylinder.

3. The scoliosis correction apparatus of claim 2, wherein the auxiliary actuator cylinder extends in a vertical direction while the main actuator cylinder extends in a horizontal direction.

4. The scoliosis correction apparatus of claim 3, wherein the main actuator cylinder has a pushing head inserted into the ball groove of the support adjuster,
wherein a first male thread is formed on an outer face of a front end of the pushing head, and a second male thread is formed on an outer face of a middle portion of the pushing head.

5. The scoliosis correction apparatus of claim 4, wherein a first female thread corresponding to the first male thread of the pushing head is formed on an inlet of the ball groove,
wherein a second female thread corresponding to the second male thread is formed on an innermost portion of the ball groove.

6. The scoliosis correction apparatus of claim 5, wherein at least one ceramic ball is formed on a top face of the foot support.

7. The scoliosis correction apparatus of claim 6, wherein the scoliosis correction apparatus further comprises a support base for supporting the foot supports, the support adjuster, the angle adjuster, and the controller thereon,
wherein the support base has a load cell configured to measure a weight of the user excluding weights of the foot support, the support adjuster, the angle adjuster, and the controller.

8. The scoliosis correction apparatus of claim 7, wherein the scoliosis correction apparatus further comprises:
a stereoscopic camera extending from the support base in a vertical direction to scan a body of the user and measure a center point of each of skeletons of the user; and
a laser irradiator for irradiating a first laser beam to an actual center point of each skeleton of the user and a second laser beam to a central point of each skeleton as a reference point for correction, thereby to induce a target posture of the user.

9. The scoliosis correction apparatus of claim 8, wherein the scoliosis correction apparatus further comprises:
a handle disposed at an upper portion of the post and moving in up and down and left and right directions and rotating around the post; and
a display connected to the handle, and moving up and down according to the up and down movement of the handle, wherein the display visually present measurements, determinations, and instructions from the controller to the user.

10. The scoliosis correction apparatus of claim 9, wherein each foot support has a plurality of acupressure protrusions projecting from a top face of the foot support and is capable of vertical movement.
